# EUROPEAN PATENT SPECIFICATION

(11) **EP 4 339 130 B1**
(45) Date of publication and mention of the grant of the patent: **02.12.2026**
(21) Application number: 22836540.9
(22) Date of filing: 25.03.2022
(51) Int. Cl.: B65G 1/04, A01N 1/145, B65G 47/90, C12M 3/00, G01N 35/00

(54) **INTELLIGENT STORAGE SYSTEM FOR SAMPLE REPOSITORY**
INTELLIGENTES SPEICHERSYSTEM FÜR PROBENSPEICHER
SYSTÈME DE STOCKAGE INTELLIGENT POUR UN DÉPÔT D'ÉCHANTILLONS

(30) Priority: 08.07.2021 CN 202110774267
(43) Date of publication of application: 20.03.2024
(73) Proprietor: SHANGHAI ORIGINCELL BIOLOGICAL CRYO EQUIPMENT CO., LTD., Shanghai 201203 (CN)
(72) Inventor: QU, Jianguo, Shanghai 201203 (CN); CAO, Weiguang, Shanghai 201203 (CN)
(74) Representative: Canzler & Bergmeier Patentanwälte Partnerschaft mbB
(86) International application number: PCT/CN2022/082897
(87) International publication number: WO 2023/279781

(56) References cited:
- WO-A2-98/43592
- CN-A- 109 368 107
- CN-A- 111 374 121
- CN-A- 113 306 943
- CN-U- 215 247 329
- FR-A1- 3 101 864
- US-B2- 9 174 790
- ANONYMOUS: "The next generation biobank system", October 2020 (2020-10-01), Internet, XP093194949, Retrieved from the Internet <URL:https://labplan.ie/content/uploads/2019/10/Askion-2020-Catalogue.pdf> [retrieved on 20240813]

## Description

### TECHNICAL FIELD

The present invention relates to the field of biological sample storage, and in particular, to an intelligent storage system for a sample repository.

### BACKGROUND

In the storage of biological samples, especially storage and extraction of cryopreservation tubes, one storage device usually corresponds to one extraction module, and samples of a plurality of devices cannot be quickly and efficiently transferred. In this case, the cost of use is increased, and the weight of a product is also significantly increased, which reduces use experience of users.

In addition, in an extraction process, a tube picking device or a storage rack grab extracts a storage rack or a sample tube from a liquid nitrogen tank and then performs transfer. In this case, the sample tube is directly exposed to room temperature, easily causing damage to a sample.

US 9174790 B2 discloses a storage system has a chamber. In the chamber, multiple storage tanks are arranged, and a picking and placing device is arranged above these tanks. Carrousels are arranged in the storage tanks. These carrousels are suspended from a top pivot bearing and laterally guided by a bottom pivot bearing. The top pivot bearing is located outside the insulation of the storage tanks and is thus protected from extreme temperatures. In the carrousel, storage cassettes are arranged which are laterally held by guide elements.

### SUMMARY OF THE INVENTION

An objective of the present invention is to provide an intelligent storage system for a sample repository, which resolves the problem that grabbing cannot be performed between a plurality of storage devices by using one type of extraction module, and samples cannot be quickly and efficiently transferred between the plurality of storage devices.

The following technical solution is adopted to resolve the technical problem in the present invention:
The first aspect of the present disclosure provides an intelligent storage system for a sample repository, which is defined in claims 1-5.

The second aspect of the present disclosure provides an intelligent storage system for a sample repository, which is defined in claims 6-10.

The present invention has the following beneficial effects:
1. Since two access ports are provided in a storage module in a face-to-face manner, and only one extraction module is required above the storage module, it is possible that two storage tanks use only one extraction module, thereby significantly improving the working efficiency and reducing the cost of use.
2. By means of a combination of two storage tanks, various types of biological samples or cells can be separately stored in one same storage module, so that cross contamination is prevented more effectively, and switching and protection can be performed in time when an accident occurs, thereby ensuring the safety of biological samples or cells.
3. The storage modules can be connected by means of multiple types of transfer devices to quickly transfer and transport samples and to provide an emergency solution. When there is a small amount of liquid nitrogen in one storage tank, samples can be transferred to a nearby storage tank, thereby minimizing a risk, and improving the working efficiency.
4. The cryopreservation tube extraction mechanism and the storage rack grabbing mechanism directly grab a cryopreservation tube and a storage rack via the access port, to provide a heat preservation solution, thereby reducing a damage probability of samples during extraction and transport.
5. The storage module can meet long term and short term storage requirements, and frequent use and long-term storage are appropriately separated, which helps to mitigate mutual impact in a storage process.

### BRIEF DESCRIPTION OF THE DRAWINGS

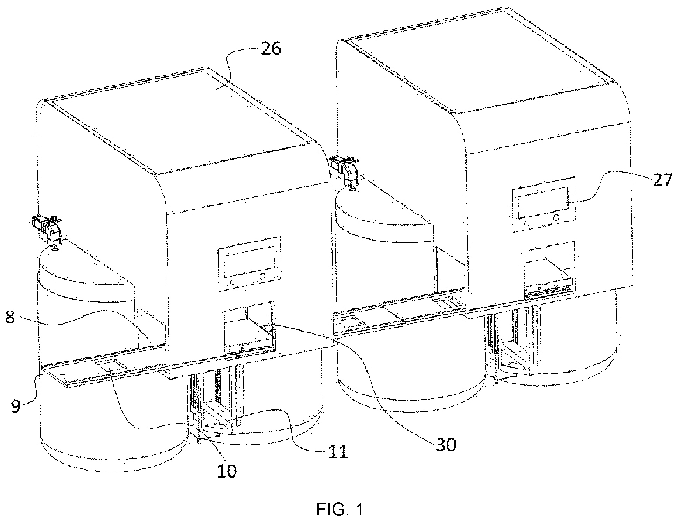

FIG. 1 is an overall schematic structural diagram according to the present invention;

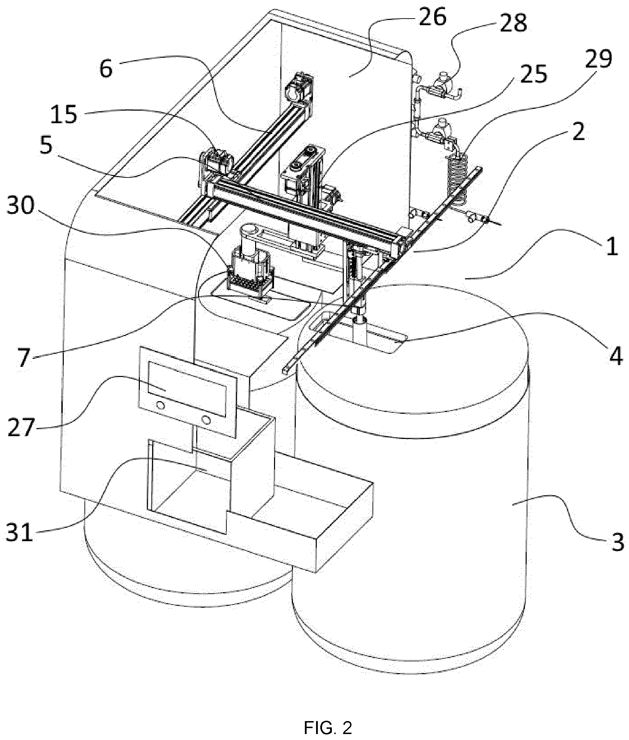

FIG. 2 is a schematic structural diagram in a module protection housing according to the present invention;

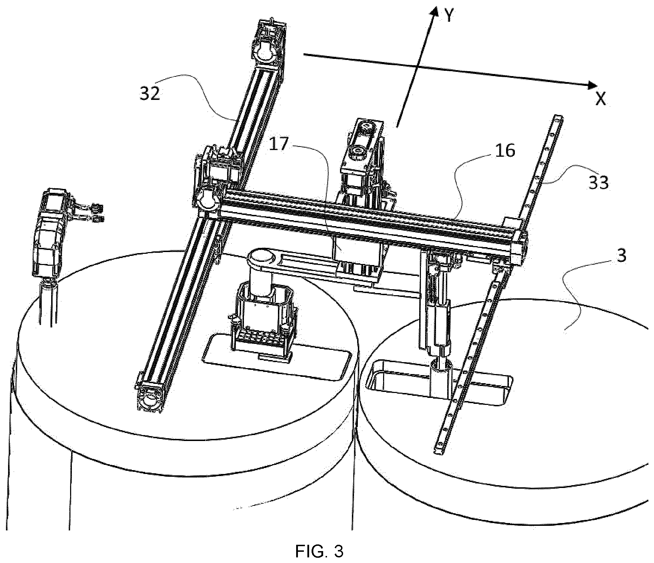

FIG. 3 is a schematic structural diagram of an extraction module according to the present invention;

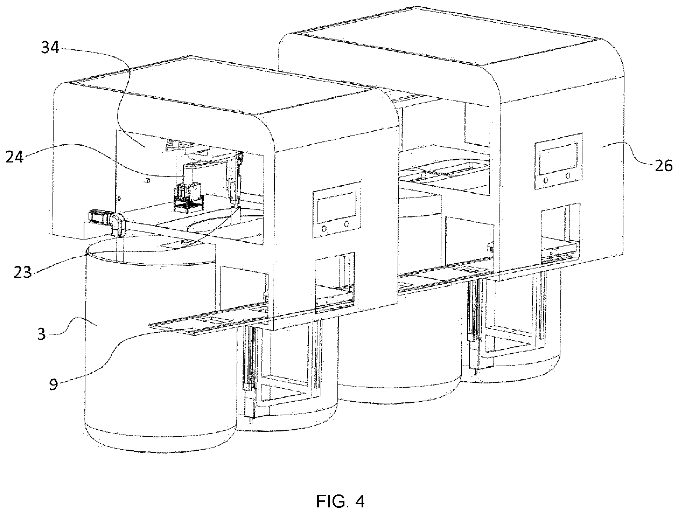

FIG. 4 is a schematic structural diagram according to Embodiment 2 of the present invention;

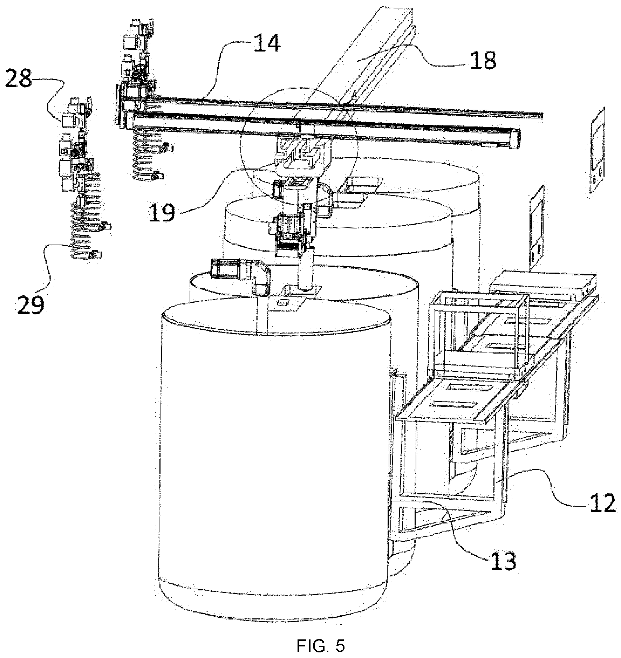

FIG. 5 is a schematic structural diagram in a module protection housing according to Embodiment 2 of the present invention; and

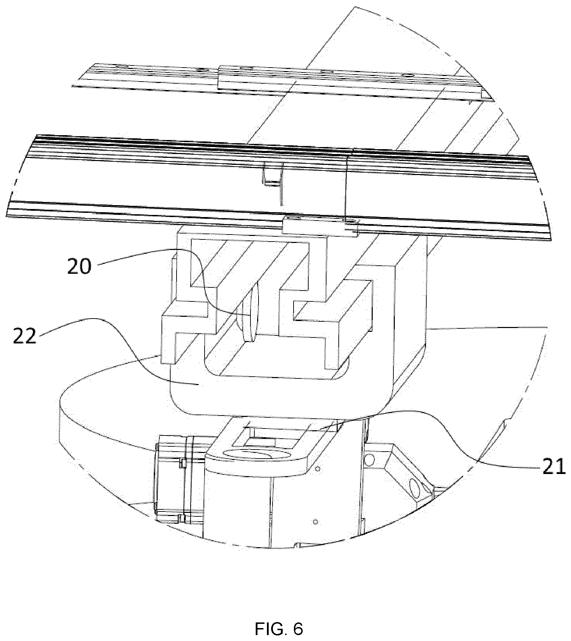

FIG. 6 is a schematic structural diagram of a sliding mechanism according to Embodiment 2 of the present invention.

### Reference numerals:

1-storage module; 2-extraction module; 3-storage tank; 4-access port; 5-X-axis extraction arm set; 6-Y-axis extraction arm set; 7-grabbing apparatus; 8-transfer channel; 9-transmission member; 10-lifting channel; H-lifting mechanism; 12-elevator platform; 13-driving electric cylinder; 14-Y-axis bearing track; 15-Y-axis movement mechanism; 16-X-axis bearing track; 17-X-axis movement mechanism; 18-X-axis sliding track; 19-sliding mechanism; 20-wheel set; 21-connecting rotating shaft; 22-sliding track bearing support; 23-cryopreservation tube extraction mechanism; 24-storage rack grabbing mechanism; 25-connecting mechanism; 26-module protection housing; 27-main control interface; 28-valve assembly; 29-coil liquid-adding mechanism; 30-transport window; 31-transport tank; 32-bearing track main shaft; 33-bearing track sub-shaft; and 34-upper transfer channel.

### DETAILED DESCRIPTION

The technical solutions of the present invention are further described below with reference to the embodiments and the accompanying drawings.

### Embodiment 1

This embodiment provides an intelligent storage system for a sample repository, including at least one storage module 1 and at least one extraction module 2.

The storage module 1 includes at least two storage tanks 3, the storage tank 3 is provided with an access port 4 in an upper end surface, and positions of the access ports 4 of the two corresponding storage tanks 3 correspond to each other.

The extraction module 2 are disposed above the storage module 1, and are capable of extracting or storing cryopreservation tubes and/or storage racks in the storage tanks 3 via the access ports 4.

During storage and extraction of a cryopreservation tube for biological samples, generally one storage tank 3 corresponds to one extraction module 2. In this case, the cost for transport between different storage tanks 3 is high, and the cryopreservation tube can be easily damaged as it is exposed to room temperature. The present invention discloses a storage module 1 consisting of two storage tanks 3. The storage tanks 3 are disposed side by side, and the access ports 4 are formed on the upper end surfaces of the storage tanks 3. The two access ports 4 are close to each other, to facilitate storage and grabbing operations of the extraction module 2 between different storage tanks 3, so that the working efficiency of the extraction module 2 can be improved, and the cost can be reduced.

In a further implementation of the present invention, the access port 4 in the storage tank 3 is formed on one side of the upper end surface of the storage tank 3, and the access ports 4 of the two storage tanks 3 that correspond to each other are opposite and close to each other. The closer the access ports 4 are to each other, the more convenient it is for the extraction module 2 to operate between different storage tanks 3, and the access ports 4 can be set face-to-face in different directions. In the present invention, the access ports 4 are set face-to-face in the radial direction and parallel to a side-by-side arrangement direction of the storage tanks 3, to maximize the convenience of the extraction module 2 in storing or extracting cryopreservation tubes on the storage module 1.

In a further implementation of the present invention, the extraction module 2 includes an X-axis extraction arm set 5, a Y-axis extraction arm set 6, and a grabbing apparatus 7. The grabbing apparatus 7 is capable of sucking or grabbing a cryopreservation tube and/or grabbing a storage rack in the storage tank 3.

The Y-axis extraction arm set 6 is connected to the X-axis extraction arm set 5, and is capable of driving the X-axis extraction arm set 5 to move along a Y axis. The X-axis extraction arm set 5 is connected to the grabbing apparatus 7, and is capable of driving the grabbing apparatus 7 to move along an X axis. The grabbing apparatus 7 can move along the X axis and the Y axis above the storage tank 3, to grab storage racks and/or cryopreservation tubes via the access ports 4 at different positions, thereby improving the working efficiency.

When the access ports 4 in the two storage tanks 3 are closer to each other, the X-axis extraction arm set 5, and the Y-axis extraction arm set 6 can grab a storage rack and/or a cryopreservation tube with fewer movements, thereby saving time and improving efficiency. Therefore, the face-to-face radial arrangement of the access ports 4 in a side-by-side direction of the storage tanks 3 has the highest efficiency.

In a further implementation of the present invention, the system includes a storage module A, an extraction module A, a storage module B, and an extraction module B.

The storage module A and the storage module B are disposed side by side, the extraction module A is disposed above and acts on the storage module A, and the extraction module is disposed above and acts on the storage module B.

A penetrating transfer channel 8 is directly provided between the extraction module A and the extraction module B, and the transfer channel 8 is capable of transferring a transport tank 31. The transport tank 31 may transit and transport a cryopreservation tube. Liquid nitrogen is stored in the transport tank 31 for heat preservation in a transport process of the cryopreservation tube.

The storage modules A and B are sequentially disposed side by side. The transfer channel 8 connects the storage module A and the storage module B in series, to quickly transfer and transport samples between different storage tanks 3.

In a further implementation of the present invention, the transfer channel 8 includes a transmission member 9 that performs transmission along a side-by-side direction of the storage module A and B.

The transmission member 9 transports the transport tank 31 in a side-by-side horizontal direction of the storage modules 1. The transmission member 9 may convey the transport tank 31 through a conveyor belt, a conveyor plate, or a conveyor track. There are many implementations of the transmission member 9, and details are not described again.

In a further implementation of the present invention, a plurality of lifting channels 10 are formed in the transmission member 9; a transport window 30 is formed in a side surface of the transfer channel 8; and the extraction module 2 further includes a lifting mechanism 11, the lifting mechanism 11 is disposed between the two storage tanks 3 of the storage module 1, and the lifting mechanism 11 is capable of lifting the transport tank 31 through the lifting channel 10.

The transport tank 31 is delivered to the transfer channel 8 from the transport window 30 formed in the side surface of the transfer channel 8, and is conveyed to an upper end of the lifting channel 10 via the transfer channel 8. The transport tank 31 is then lifted by the lifting mechanism 11 to an upper end of the storage tank 3 through the lifting channel 10, to store or extract a cryopreservation tube. After operations are ended, the lifting mechanism 11 descends to deliver the transport tank 31 back to the transfer channel 8. A cryopreservation tube in the transport tank 31 can be quickly transported and transferred between different storage modules 1 by the transmission member 9 in the transfer channel 8, thereby improving the working efficiency.

In a further implementation of the present invention, the lifting mechanism 11 includes an elevator platform 12 and a driving electric cylinder 13. The elevator platform 12 is disposed between the two storage tanks 3 of the storage module 1, one end of the elevator platform 12 is disposed below the lifting channel 10, the other end of the elevator platform 12 extends outward and is vertically disposed, and the driving electric cylinder 13 is vertically disposed and is connected to an extension end of the elevator platform 12. The driving electric cylinder 13 is disposed on a side of the transfer channel 8 close to the storage tank 3, so that the overall space of this system can be reduced. The driving electric cylinder 13 is capable of driving the elevator platform 12 to ascend or descend in a vertical direction. The lifting mechanism 11 resolves the problem of transport between the storage tank 3 and the transfer channel 8, thereby significantly improving the efficiency of quickly transferring samples.

In a further implementation of the present invention, the Y-axis extraction arm set 6 includes a Y-axis bearing track 14 and a Y-axis movement mechanism 15. The Y-axis bearing track 14 includes a bearing track main shaft 32 and a bearing track sub-shaft 33. The bearing track main shaft 32 and the bearing track sub-shaft 33 are disposed in parallel and symmetrically above two sides of the storage module 1, the Y-axis movement mechanism 15 is disposed on the bearing track main shaft 32 and the bearing track sub-shaft 33 and is capable of sliding along the bearing track main shaft 32 and the bearing track sub-shaft 33, and the Y-axis movement mechanism 15 is connected to the X-axis extraction arm set 5 and is capable of driving the X-axis extraction arm set 5 to move along the Y axis direction. The Y-axis is a horizontal direction perpendicular to the direction in which the storage tanks 3 are set side by side.

The Y-axis extraction arm set 6 is used to transport a cryopreservation tube between the transport tank 31 and the storage tank 3. The grabbing apparatus 7 is driven by the Y-axis extraction arm set 6 to move in the horizontal direction perpendicular to the direction in which the storage tanks 3 are set side by side, to facilitate the grabbing operations of the grabbing apparatus.

The Y-axis movement mechanism 15 is provided with a strap to be fastened to the X-axis extraction arm set 5, and is provided with a sliding member. While sliding on the bearing track main shaft 32 and the bearing track sub-shaft 33, the Y-axis movement mechanism drives the X-axis extraction arm set 5 to move horizontally along the Y axis, and the Y-axis movement mechanism 15 may be implemented as a sliding block, a sliding wheel, or the like.

In a further implementation of the present invention, the X-axis extraction arm set 5 includes an X-axis bearing track 16 and an X-axis movement mechanism 17. Two ends of the X-axis bearing track 16 are vertically disposed on the bearing track main shaft 32 and the bearing track sub-shaft 33 and are connected to the Y-axis movement mechanism 15. The X-axis movement mechanism 17 is disposed on the X-axis bearing track 16 and is capable of sliding along the X-axis bearing track 16, and the X-axis movement mechanism 17 is connected to the grabbing apparatus 7 and is capable of driving the grabbing apparatus 7 to move in an X axis direction. The X axis is parallel to the direction in which the storage tanks 3 are set side by side.

The two ends of the X-axis bearing track 16 are respectively disposed on the bearing track main shaft 32 and the bearing track sub-shaft 33 to move horizontally in one storage module 1, and the grabbing apparatus 7 can be driven to move in an X axis direction, that is, to move between the access ports 4 of the two storage tanks 3, thereby performing grabbing and storage operations.

In a further implementation of the present invention, the grabbing apparatus 7 includes a cryopreservation tube extraction mechanism 23, a storage rack grabbing mechanism 24, and a connecting mechanism 25; an upper end of the connecting mechanism 25 is connected to the X-axis extraction arm set 5, and a lower end of the connecting mechanism 25 is connected to the storage rack grabbing mechanism 24 and the cryopreservation tube extraction mechanism 23.

The X-axis extraction arm set 5 is capable of grabbing a cryopreservation tube or a storage rack in the storage tank 3 and is capable of driving the cryopreservation tube or the storage rack to move along the X axis to grab the cryopreservation tube and/or the storage rack, to transport samples between different storage tanks 3; and the Y-axis extraction arm set 6 is capable of grabbing a cryopreservation tube or a storage rack in the storage tank 3 and is capable of driving the cryopreservation tube or the storage rack to move along the Y axis to transport samples between the storage tank 3 and the transport tank 31, to transport samples between different storage modules 1, thereby significantly improving the working efficiency.

In a further implementation of the present invention, the system further includes a module protection housing 26. The module protection housing 26 is disposed above the storage module 1 and covers the extraction module 2. Two ends of the Y-axis bearing track 14 are both fastened on an inner wall of the module protection housing 26, and the lifting mechanism 11 is fastened at a lower end of the module protection housing 26. The module protection housing 26 fastens the extraction module 2, and protects a cryopreservation tube extracted by the grabbing apparatus 7 from losing heat too quickly, thereby implementing heat preservation.

A main control interface 27 is further provided on the module protection housing 26. The main control interface 27 is disposed on another side of the transfer channel 8 opposite to the storage tank 3. The main control interface 27 may control the transmission member 9 to start or stop transferring the transport tank 31.

In a further implementation of the present invention, the storage module 1 further includes a valve assembly 28. The valve assembly 28 is disposed on a side surface of the module protection housing 26. The valve assembly 28 may inject liquid nitrogen into the storage tank 3, to maintain a temperature in the tank.

In a further implementation of the present invention, the valve assembly 28 includes a coil liquid-adding mechanism 29. The coil liquid-adding mechanism 29 is disposed on the side surface of the module protection housing 26. The coil liquid-adding mechanism 29 is heated to inject sprayed liquid nitrogen into the storage tank 3, to keep liquid nitrogen from being directly poured at one position to cause temperature non-uniformity. If the temperature in the storage tank 3 is excessively low, a heating wire outside the coil liquid-adding mechanism 29 may be turned on to reach an appropriate storage temperature.

### Embodiment 2

This embodiment provides an intelligent storage system for a sample repository. This embodiment differs from Embodiment 2 in that:

The X-axis extraction arm set 5 includes an X-axis sliding track 18 and a sliding mechanism 19. The X-axis sliding track 18 is vertically disposed at lower ends of the bearing track main shaft 32 and the bearing track sub-shaft 33, with two ends extending to connect the storage module A and the storage module B in series. The X-axis sliding track 18 is connected to the Y-axis movement mechanism 15, the sliding mechanism 19 is disposed on the X-axis sliding track 18 and is capable of sliding along the X-axis sliding track 18, and the sliding mechanism 19 is connected to the grabbing apparatus 7 and is capable of driving the grabbing apparatus 7 to move in an X axis direction. The X axis is parallel to the direction in which the storage tanks 3 are set side by side.

Compared with that the X-axis bearing track 16 moves horizontally along the Y-axis bearing track 14 in Embodiment 1, two ends of the X-axis sliding track 18 extend and may connect in series all the storage module A and the storage module B that are disposed side by side, to transfer samples by multiple types of transfer devices between different storage modules 1, thereby significantly improving the transfer efficiency.

In a further implementation of the present invention, the sliding mechanism 19 includes a wheel set 20, a connecting rotating shaft 21, and a sliding track bearing support 22. The wheel set 20 is symmetrically disposed in the X-axis sliding track 18 and is capable of sliding along the X-axis sliding track 18, the sliding track bearing support 22 is disposed on the X-axis sliding track 18 and is connected to the wheel set 20, and the connecting rotating shaft 21 is disposed on the sliding track bearing support 22 and is connected to the grabbing apparatus 7.

The sliding track bearing support 22 slides on the X-axis sliding track 18 through the wheel set 20, a lower end of the connecting rotating shaft 21 is connected to the grabbing apparatus 7 to drive the grabbing apparatus 7 to move along the X-axis sliding track 18, to implement grabbing and storage between different storage modules 1 by the grabbing apparatus 7.

In a further implementation of the present invention, an upper transfer channel 34 is provided in the module protection housing 26. The upper transfer channel 34 is provided above the storage tank 3 and accommodates the extraction module 2. The upper transfer channel 34 and the transfer channel 8 are provided in the same direction. The X-axis sliding track 18 passes through the upper transfer channel 34 to drive the grabbing apparatus 7 to transport samples between different storage modules 1, thereby significantly improving the working efficiency.

The foregoing sequence of the embodiments are merely for the convenience of description, and do not imply the preference among the embodiments.

Finally, it should be noted that the foregoing embodiments are merely intended for describing the technical solutions of the present invention rather than limiting the present invention. Although the present invention is described in detail with reference to the foregoing embodiments, persons of ordinary skill in the art should understand that they may still make modifications to the technical solutions described in the foregoing embodiments or make equivalent replacements to some technical features thereof, without departing from the scope of the present invention as defined by the claims.

## Claims

1. An intelligent storage system for a sample repository, comprising storage modules (1) and extraction modules (2), wherein
each storage module (1) comprises at least two storage tanks (3), each of the storage tanks (3) is provided with an access port (4) in an upper end surface, and access ports (4) of two storage tanks (3) are opposite and close to each other; each storage module (1) is associated with one extraction module (2); the extraction module (2) is disposed above the corresponding storage module (1), and is configured to extract or store cryopreservation tubes and/or storage racks in the storage tanks (3) via the access ports (4);
wherein the intelligent storage system comprises a storage module A (1), an extraction module A (2), a storage module B (1), and an extraction module B (2), said four storage tanks (3) of said two storage modules (1) form a collinear arrangement, wherein the storage module A (1) and the storage module B (1) are disposed side by side, the extraction module A (2) is disposed above and acts on the storage module A (1), and the extraction module B (2) is disposed above and acts on the storage module B (1);
wherein a module protection housing (26) is disposed above each storage module (1) and covers each respective extraction module (2), and a main control interface (27) is provided on each module protection housing (26);
a penetrating transfer channel (8) penetrates through the module protection housings (26) and is provided laterally along the storage module A (1) and storage module B (1), and the penetrating transfer channel (8) is configured to transfer a transport tank (31) containing liquid nitrogen; the penetrating transfer channel (8) comprises a transmission member (9) for performing transmission through the penetrating transfer channel (8), a plurality of lifting channels (10) are formed in the transmission member (9); and
each of the extraction modules (2) further comprises a lifting mechanism (11), the lifting mechanism (11) is disposed between two storage tanks (3) of each of the storage modules (1), and the lifting mechanism (11) is configured to lift the transport tank (31) through the plurality of lifting channels (10).

2. The intelligent storage system for a sample repository according to claim 1, wherein each of the extraction modules (2) comprises an X-axis extraction arm set (5), a Y-axis extraction arm set (6), and a grabbing apparatus (7), wherein the grabbing apparatus (7) is capable of sucking or grabbing a cryopreservation tube and/or grabbing a storage rack in the storage tanks (3); and
the X-axis extraction arm set (5) is capable of driving the grabbing apparatus (7) to move along an X axis, and the Y-axis extraction arm set (6) is capable of driving the grabbing apparatus (7) to move along a Y axis.

3. The intelligent storage system for a sample repository according to claim 1, wherein the lifting mechanism (11) comprises an elevator platform (12) and a driving electric cylinder (13), the elevator platform (12) is disposed between the two storage tanks (3) of each of the storage modules (1), one end of the elevator platform (12) is disposed below the lifting channels (10), the other end of the elevator platform (12) extends outward and is vertically disposed, and the driving electric cylinder (13) is vertically disposed and is connected to an extension end of the elevator platform (12); and
the driving electric cylinder (13) is capable of driving the elevator platform (12) to ascend or descend in a vertical direction.

4. The intelligent storage system for a sample repository according to claim 2 or 3, wherein the Y-axis extraction arm set (6) comprises a Y-axis bearing track (14) and a Y-axis movement mechanism (15), and the Y-axis bearing track (14) comprises a bearing track main shaft (32) and a bearing track sub-shaft (33);
the bearing track main shaft (32) and the bearing track sub-shaft (33) are disposed in parallel and symmetrically above two sides of one of the storage modules (1), the Y-axis movement mechanism (15) is disposed on the bearing track main shaft (32) and the bearing track sub-shaft (33) and is capable of sliding along the bearing track main shaft (32) and the bearing track sub-shaft (33), and the Y-axis movement mechanism (15) is connected to the X-axis extraction arm set (5) and is capable of driving the X-axis extraction arm set (5) to move along the Y axis direction; and
the Y axis is perpendicular to a direction along which the storage tanks (3) are set side by side.

5. The intelligent storage system for a sample repository according to claim 4, wherein the X-axis extraction arm set (5) comprises an X-axis bearing track (16) and an X-axis movement mechanism (17);
two ends of the X-axis bearing track (16) are vertically disposed on the Y-axis bearing track (14) and are connected to the Y-axis movement mechanism (15), the X-axis movement mechanism (17) is disposed on the X-axis bearing track (16) and is capable of sliding along the X-axis bearing track (16), and the X-axis movement mechanism (17) is connected to the grabbing apparatus (7) and is capable of driving the grabbing apparatus (7) to move in an X axis direction; and
the X axis is parallel to a direction along which the storage tanks (3) are set side by side.

6. An intelligent storage system for a sample repository, comprising:
two storage modules (1), each storage module (1) comprising at least two storage tanks (3) arranged side by side, each storage tank (3) is provided with an access port (4) in an upper end surface, and access ports (4) of two storage tanks (3) are opposite and close to each other; and
an extraction module (2) disposed above the storage modules (1) and configured to extract or store cryopreservation tubes and/or storage racks in the storage tanks (3) through the access ports (4);
wherein each storage module (1) is provided with a module protection housing (26), the extraction module (2) is arranged within the module protection housings (26);
wherein the extraction module (2) comprises an X-axis extraction arm set (5), a Y-axis extraction arm set (6), and a grabbing apparatus (7), wherein
the grabbing apparatus (7) is configured to grasp or suck a cryopreservation tube and/or a storage rack;
the Y-axis extraction arm set (6) comprises a Y-axis bearing track (14) and a Y-axis movement mechanism (15), the Y-axis movement mechanism (15) is movable along the Y-axis bearing track (14) and connected to the X-axis extraction arm set (5) to drive the X-axis extraction arm set (5) to move in a Y-axis direction; and
the X-axis extraction arm set (5) comprises an X-axis sliding track (18) and a sliding mechanism (19), the sliding mechanism (19) is movable along the X-axis sliding track (18) and connected to the grabbing apparatus (7); wherein the X-axis sliding track (18) extends through and between the module protection housings (26) and connects the storage modules (1) such that the grabbing apparatus (7) is movable between the storage modules (1) by means of the X-axis sliding track (18), to allow the extraction module (2) to access storage tanks of different storage modules (1);
wherein a penetrating transfer channel (8) penetrates through the module protection housings (26) and is provided laterally along the storage modules (1) and is configured to transfer a transport tank (31) containing liquid nitrogen; and
wherein the penetrating transfer channel (8) comprises a transmission member (9) for transmission through the penetrating transfer channel, provided with a plurality of lifting channels (10), and a lifting mechanism (11) is disposed between two storage tanks (3) of each storage module (1) and configured to lift the transport tank (31) through the lifting channels (10).

7. The intelligent storage system for a sample repository according to claim 6, wherein the sliding mechanism (19) comprises a wheel set (20), a connecting rotating shaft (21), and a sliding track bearing support (22); and
the wheel set (20) is symmetrically disposed in the X-axis sliding track (18) and is capable of sliding along the X-axis sliding track (18), the sliding track bearing support (22) is disposed on the X-axis sliding track (18) and is connected to the wheel set (20), and the connecting rotating shaft (21) is disposed on the sliding track bearing support (22) and is connected to the grabbing apparatus (7).

8. The intelligent storage system for a sample repository according to claim 5, 6, or 7, wherein the grabbing apparatus (7) comprises a cryopreservation tube extraction mechanism (23), a storage rack grabbing mechanism (24), and a connecting mechanism (25);
an upper end of the connecting mechanism (25) is connected to the X-axis extraction arm set (5), and a lower end of the connecting mechanism (25) is connected to the storage rack grabbing mechanism (24) and the cryopreservation tube extraction mechanism (23); and
the X-axis extraction arm set (5) is capable of grabbing a cryopreservation tube or a storage rack in the storage tank and is capable of driving the cryopreservation tube or the storage rack to move along the X axis, and the Y-axis extraction arm set (6) is capable of allowing for grabbing a cryopreservation tube or a storage rack in the storage tank and is capable of driving the cryopreservation tube or the storage rack to move along the Y axis.

9. The intelligent storage system for a sample repository according to claim 8, wherein each of the storage modules (1) further comprises a valve assembly (28), and the valve assembly (28) is disposed on a side surface of the module protection housing (26).

10. The intelligent storage system for a sample repository according to claim 9, wherein the valve assembly (28) comprises a coil liquid-adding mechanism (29), and the coil liquid-adding mechanism (29) is disposed on a side surface of the module protection housing (26).

## Patentansprüche

1. Intelligentes Lagersystem für ein Probenrepositorium, umfassend Lagermodule (1) und Entnahmemodule (2), wobei
jedes Lagermodul (1) mindestens zwei Lagerbehälter (3) umfasst, jeder der Lagerbehälter (3) mit einem Zugriffsanschluss (4) in einer oberen Endoberfläche versehen ist und die Zugriffsanschlüsse (4) von zwei Lagerbehältern (3) einander gegenüberliegen und nahe beieinander liegen; jedes Lagermodul (1) einem Entnahmemodul (2) zugeordnet ist; das Entnahmemodul (2) über dem entsprechenden Lagermodul (1) angeordnet ist und konfiguriert ist, um Kryokonservierungsröhrchen und/oder Lagergestelle in den Lagerbehältern (3) über die Zugriffsanschlüsse (4) zu entnehmen oder zu lagern;
wobei das intelligente Lagersystem ein Lagermodul A (1), ein Entnahmemodul A (2), ein Lagermodul B (1) und ein Entnahmemodul B (2) umfasst, die vier genannten Lagerbehälter (3) der zwei genannten Lagermodule (1) eine kollineare Anordnung bilden, wobei das Lagermodul A (1) und das Lagermodul B (1) nebeneinander angeordnet sind, das Entnahmemodul A (2) über dem Lagermodul A (1) angeordnet ist und auf dieses wirkt und das Entnahmemodul B (2) über dem Lagermodul B (1) angeordnet ist und auf dieses wirkt;
wobei ein Modulschutzgehäuse (26) über jedem Lagermodul (1) angeordnet ist und jedes jeweilige Entnahmemodul (2) abdeckt und eine Hauptsteuerungsschnittstelle (27) an jedem Modulschutzgehäuse (26) bereitgestellt ist;
ein durchdringender Übertragungskanal (8) die Modulschutzgehäuse (26) durchdringt und seitlich entlang des Lagermoduls A (1) und des Lagermoduls B (1) bereitgestellt ist und der durchdringende Übertragungskanal (8) konfiguriert ist, um einen Transportbehälter (31), der Flüssigstickstoff beinhaltet, zu übertragen; der durchdringende Übertragungskanal (8) ein Übertragungselement (9) zum Durchführen einer Übertragung durch den durchdringenden Übertragungskanal (8) umfasst, eine Vielzahl von Hebekanälen (10) in dem Übertragungselement (9) gebildet ist; und
jedes der Entnahmemodule (2) ferner einen Hebemechanismus (11) umfasst, der Hebemechanismus (11) zwischen zwei Lagerbehältern (3) von jedem der Lagermodule (1) angeordnet ist und der Hebemechanismus (11) konfiguriert ist, um den Transportbehälter (31) durch die Vielzahl von Hebekanälen (10) zu heben.

2. Intelligentes Lagersystem für ein Probenrepositorium nach Anspruch 1, wobei jedes der Entnahmemodule (2) einen X-Achsen-Entnahme-Arm-Satz (5), einen Y-Achsen-Entnahme-Arm-Satz (6) und eine Greifvorrichtung (7) umfasst, wobei die Greifvorrichtung (7) in der Lage ist, ein Kryokonservierungsröhrchen anzusaugen oder zu greifen und/oder ein Lagergestell in den Lagerbehältern (3) zu greifen; und
der X-Achsen-Entnahme-Arm-Satz (5) in der Lage ist, die Greifvorrichtung (7) anzutreiben, um sich entlang einer X-Achse zu bewegen, und der Y-Achsen-Entnahme-Arm-Satz (6) in der Lage ist, die Greifvorrichtung (7) anzutreiben, um sich entlang einer Y-Achse zu bewegen.

3. Intelligentes Lagersystem für ein Probenrepositorium nach Anspruch 1, wobei der Hebemechanismus (11) eine Aufzugplattform (12) und einen antreibenden Elektrozylinder (13) umfasst, die Aufzugplattform (12) zwischen den zwei Lagerbehältern (3) von jedem der Lagermodule (1) angeordnet ist, ein Ende der Aufzugplattform (12) unter den Hebekanälen (10) angeordnet ist, das andere Ende der Aufzugplattform (12) sich nach außen erstreckt und vertikal angeordnet ist und der antreibende Elektrozylinder (13) vertikal angeordnet ist und mit einem Verlängerungsende der Aufzugplattform (12) verbunden ist; und
der antreibende Elektrozylinder (13) in der Lage ist, die Aufzugplattform (12) anzutreiben, um in einer vertikalen Richtung aufzusteigen oder abzusteigen.

4. Intelligentes Lagersystem für ein Probenrepositorium nach Anspruch 2 oder 3, wobei der Y-Achsen-Entnahme-Arm-Satz (6) eine Y-Achsen-Lagerbahn (14) und einen Y-Achsen-Bewegungsmechanismus (15) umfasst und die Y-Achsen-Lagerbahn (14) eine Lagerbahn-Hauptwelle (32) und eine Lagerbahn-Nebenwelle (33) umfasst;
die Lagerbahn-Hauptwelle (32) und die Lagerbahn-Nebenwelle (33) parallel und symmetrisch über zwei Seiten von einem der Lagermodule (1) angeordnet sind, der Y-Achsen-Bewegungsmechanismus (15) an der Lagerbahn-Hauptwelle (32) und der Lagerbahn-Nebenwelle (33) angeordnet ist und in der Lage ist, entlang der Lagerbahn-Hauptwelle (32) und der Lagerbahn-Nebenwelle (33) zu gleiten, und der Y-Achsen-Bewegungsmechanismus (15) mit dem X-Achsen-Entnahme-Arm-Satz (5) verbunden ist und in der Lage ist, den X-Achsen-Entnahme-Arm-Satz (5) anzutreiben, um sich entlang der Y-Achsen-Richtung zu bewegen; und
die Y-Achse senkrecht zu einer Richtung ist, entlang der die Lagerbehälter (3) nebeneinander angeordnet sind.

5. Intelligentes Lagersystem für ein Probenrepositorium nach Anspruch 4, wobei der X-Achsen-Entnahme-Arm-Satz (5) eine X-Achsen-Lagerbahn (16) und einen X-Achsen-Bewegungsmechanismus (17) umfasst;
zwei Enden der X-Achsen-Lagerbahn (16) vertikal an der Y-Achsen-Lagerbahn (14) angeordnet sind und mit dem Y-Achsen-Bewegungsmechanismus (15) verbunden sind, der X-Achsen-Bewegungsmechanismus (17) an der X-Achsen-Lagerbahn (16) angeordnet ist und in der Lage ist, entlang der X-Achsen-Lagerbahn (16) zu gleiten, und der X-Achsen-Bewegungsmechanismus (17) mit der Greifvorrichtung (7) verbunden ist und in der Lage ist, die Greifvorrichtung (7) anzutreiben, um sich in einer X-Achsen-Richtung zu bewegen; und
die X-Achse parallel zu einer Richtung ist, entlang der die Lagerbehälter (3) nebeneinander angeordnet sind.

6. Intelligentes Lagersystem für ein Probenrepositorium, umfassend:
zwei Lagermodule (1), wobei jedes Lagermodul (1) mindestens zwei nebeneinander angeordnete Lagerbehälter (3) umfasst, jeder Lagerbehälter (3) mit einem Zugriffsanschluss (4) in einer oberen Endoberfläche versehen ist und die Zugriffsanschlüsse (4) von zwei Lagerbehältern (3) einander gegenüberliegen und nahe beieinander liegen; und
ein Entnahmemodul (2), das über den Lagermodulen (1) angeordnet ist und konfiguriert ist, um Kryokonservierungsröhrchen und/oder Lagergestelle in den Lagerbehältern (3) durch die Zugriffsanschlüsse (4) zu entnehmen oder zu lagern;
wobei jedes Lagermodul (1) mit einem Modulschutzgehäuse (26) versehen ist, das Entnahmemodul (2) innerhalb der Modulschutzgehäuse (26) angeordnet ist;
wobei das Entnahmemodul (2) einen X-Achsen-Entnahme-Arm-Satz (5), einen Y-Achsen-Entnahme-Arm-Satz (6) und eine Greifvorrichtung (7) umfasst, wobei
die Greifvorrichtung (7) konfiguriert ist, um ein Kryokonservierungsröhrchen und/oder ein Lagergestell zu greifen oder anzusaugen;
der Y-Achsen-Entnahme-Arm-Satz (6) eine Y-Achsen-Lagerbahn (14) und einen Y-Achsen-Bewegungsmechanismus (15) umfasst, der Y-Achsen-Bewegungsmechanismus (15) entlang der Y-Achsen-Lagerbahn (14) bewegbar ist und mit dem X-Achsen-Entnahme-Arm-Satz (5) verbunden ist, um den X-Achsen-Entnahme-Arm-Satz (5) anzutreiben, um sich in einer Y-Achsen-Richtung zu bewegen; und
der X-Achsen-Entnahme-Arm-Satz (5) eine X-Achsen-Gleitbahn (18) und einen Gleitmechanismus (19) umfasst, der Gleitmechanismus (19) entlang der X-Achsen-Gleitbahn (18) bewegbar ist und mit der Greifvorrichtung (7) verbunden ist; wobei sich die X-Achsen-Gleitbahn (18) durch und zwischen den Modulschutzgehäusen (26) erstreckt und die Lagermodule (1) derart verbindet, dass die Greifvorrichtung (7) zwischen den Lagermodulen (1) mittels der X-Achsen-Gleitbahn (18) bewegbar ist, um dem Entnahmemodul (2) zu ermöglichen, auf Lagerbehälter von unterschiedlichen Lagermodulen (1) zuzugreifen;
wobei ein durchdringender Übertragungskanal (8) die Modulschutzgehäuse (26) durchdringt und seitlich entlang der Lagermodule (1) bereitgestellt ist und konfiguriert ist, um einen Transportbehälter (31), der Flüssigstickstoff beinhaltet, zu übertragen; und
wobei der durchdringende Übertragungskanal (8) ein Übertragungselement (9) zur Übertragung durch den durchdringenden Übertragungskanal umfasst, das mit einer Vielzahl von Hebekanälen (10) versehen ist, und ein Hebemechanismus (11) zwischen zwei Lagerbehältern (3) von jedem Lagermodul (1) angeordnet ist und konfiguriert ist, um den Transportbehälter (31) durch die Hebekanäle (10) zu heben.

7. Intelligentes Lagersystem für ein Probenrepositorium nach Anspruch 6, wobei der Gleitmechanismus (19) einen Radsatz (20), eine verbindende Drehwelle (21) und eine Gleitbahn-Lagerstütze (22) umfasst; und
der Radsatz (20) symmetrisch in der X-Achsen-Gleitbahn (18) angeordnet ist und in der Lage ist, entlang der X-Achsen-Gleitbahn (18) zu gleiten, die Gleitbahn-Lagerstütze (22) an der X-Achsen-Gleitbahn (18) angeordnet ist und mit dem Radsatz (20) verbunden ist und die verbindende Drehwelle (21) an der Gleitbahn-Lagerstütze (22) angeordnet ist und mit der Greifvorrichtung (7) verbunden ist.

8. Intelligentes Lagersystem für ein Probenrepositorium nach Anspruch 5, 6 oder 7, wobei die Greifvorrichtung (7) einen Kryokonservierungsröhrchen-Entnahmemechanismus (23), einen Lagergestell-Greifmechanismus (24) und einen Verbindungsmechanismus (25) umfasst;
ein oberes Ende des Verbindungsmechanismus (25) mit dem X-Achsen-Entnahme-Arm-Satz (5) verbunden ist und ein unteres Ende des Verbindungsmechanismus (25) mit dem Lagergestell-Greifmechanismus (24) und dem Kryokonservierungsröhrchen-Entnahmemechanismus (23) verbunden ist; und
der X-Achsen-Entnahme-Arm-Satz (5) in der Lage ist, ein Kryokonservierungsröhrchen oder ein Lagergestell in dem Lagerbehälter zu greifen, und in der Lage ist, das Kryokonservierungsröhrchen oder das Lagergestell anzutreiben, um sich entlang der X-Achse zu bewegen, und der Y-Achsen-Entnahme-Arm-Satz (6) in der Lage ist, das Greifen eines Kryokonservierungsröhrchens oder eines Lagergestells in dem Lagerbehälter zu ermöglichen, und in der Lage ist, das Kryokonservierungsröhrchen oder das Lagergestell anzutreiben, um sich entlang der Y-Achse zu bewegen.

9. Intelligentes Lagersystem für ein Probenrepositorium nach Anspruch 8, wobei jedes der Lagermodule (1) ferner eine Ventilbaugruppe (28) umfasst und die Ventilbaugruppe (28) an einer Seitenoberfläche des Modulschutzgehäuses (26) angeordnet ist.

10. Intelligentes Lagersystem für ein Probenrepositorium nach Anspruch 9, wobei die Ventilbaugruppe (28) einen Spulen-Flüssigkeitszugabemechanismus (29) umfasst und der Spulen-Flüssigkeitszugabemechanismus (29) an einer Seitenoberfläche des Modulschutzgehäuses (26) angeordnet ist.

## Revendications

1. Système de stockage intelligent pour un référentiel d'échantillons, comprenant des modules de stockage (1) et des modules d'extraction (2), dans lequel
chaque module de stockage (1) comprend au moins deux réservoirs de stockage (3), chacun des réservoirs de stockage (3) est pourvu d'un port d'accès (4) dans une surface d'extrémité supérieure, et les ports d'accès (4) de deux réservoirs de stockage (3) sont opposés et proches l'un de l'autre ; chaque module de stockage (1) est associé à un module d'extraction (2) ; le module d'extraction (2) est disposé au-dessus du module de stockage correspondant (1), et est configuré pour extraire ou stocker des tubes de cryoconservation et/ou des racks de stockage dans les réservoirs de stockage (3) via les ports d'accès (4) ;
dans lequel le système de stockage intelligent comprend un module de stockage A (1), un module d'extraction A (2), un module de stockage B (1) et un module d'extraction B (2), lesdits quatre réservoirs de stockage (3) desdits deux modules de stockage (1) forment un agencement colinéaire, dans lequel le module de stockage A (1) et le module de stockage B (1) sont disposés côte à côte, le module d'extraction A (2) est disposé au-dessus et agit sur le module de stockage A (1), et le module d'extraction B (2) est disposé au-dessus et agit sur le module de stockage B (1) ;
dans lequel un boîtier de protection de module (26) est disposé au-dessus de chaque module de stockage (1) et recouvre chaque module d'extraction respectif (2), et une interface de commande principale (27) est prévue sur chaque boîtier de protection de module (26) ;
un canal de transfert pénétrant (8) pénètre à travers les boîtiers de protection de module (26) et est prévu latéralement le long du module de stockage A (1) et du module de stockage B (1), et le canal de transfert pénétrant (8) est configuré pour transférer un réservoir de transport (31) contenant de l'azote liquide ; le canal de transfert pénétrant (8) comprend un élément de transmission (9) pour réaliser une transmission à travers le canal de transfert pénétrant (8), une pluralité de canaux de levage (10) sont formés dans l'élément de transmission (9) ; et
chacun des modules d'extraction (2) comprend en outre un mécanisme de levage (11), le mécanisme de levage (11) est disposé entre deux réservoirs de stockage (3) de chacun des modules de stockage (1), et le mécanisme de levage (11) est configuré pour lever le réservoir de transport (31) à travers la pluralité de canaux de levage (10).

2. Système de stockage intelligent pour un référentiel d'échantillons selon la revendication 1, dans lequel chacun des modules d'extraction (2) comprend un ensemble de bras d'extraction d'axe X (5), un ensemble de bras d'extraction d'axe Y (6) et un appareil de saisie (7), dans lequel l'appareil de saisie (7) est capable d'aspirer ou de saisir un tube de cryoconservation et/ou de saisir un rack de stockage dans les réservoirs de stockage (3) ; et
l'ensemble de bras d'extraction d'axe X (5) est capable d'entraîner l'appareil de saisie (7) à se déplacer le long d'un axe X, et l'ensemble de bras d'extraction d'axe Y (6) est capable d'entraîner l'appareil de saisie (7) à se déplacer le long d'un axe Y.

3. Système de stockage intelligent pour un référentiel d'échantillons selon la revendication 1, dans lequel le mécanisme de levage (11) comprend une plate-forme d'ascenseur (12) et un cylindre électrique d'entraînement (13), la plate-forme d'ascenseur (12) est disposée entre les deux réservoirs de stockage (3) de chacun des modules de stockage (1), une extrémité de la plate-forme d'ascenseur (12) est disposée sous les canaux de levage (10), l'autre extrémité de la plate-forme d'ascenseur (12) s'étend vers l'extérieur et est disposée verticalement, et le cylindre électrique d'entraînement (13) est disposé verticalement et est relié à une extrémité d'extension de la plate-forme d'ascenseur (12) ; et
le cylindre électrique d'entraînement (13) est capable d'entraîner la plate-forme d'ascenseur (12) à monter ou à descendre dans une direction verticale.

4. Système de stockage intelligent pour un référentiel d'échantillons selon la revendication 2 ou 3, dans lequel l'ensemble de bras d'extraction d'axe Y (6) comprend une piste de palier d'axe Y (14) et un mécanisme de mouvement d'axe Y (15), et la piste de palier d'axe Y (14) comprend un arbre principal de piste de palier (32) et un sous-arbre de piste de palier (33) ;
l'arbre principal de piste de palier (32) et le sous-arbre de piste de palier (33) sont disposés en parallèle et symétriquement au-dessus de deux côtés de l'un des modules de stockage (1), le mécanisme de mouvement d'axe Y (15) est disposé sur l'arbre principal de piste de palier (32) et le sous-arbre de piste de palier (33) et est capable de coulisser le long de l'arbre principal de piste de palier (32) et du sous-arbre de piste de palier (33), et le mécanisme de mouvement d'axe Y (15) est relié à l'ensemble de bras d'extraction d'axe X (5) et est capable d'entraîner l'ensemble de bras d'extraction d'axe X (5) à se déplacer le long de la direction de l'axe Y ; et
l'axe Y est perpendiculaire à une direction le long de laquelle les réservoirs de stockage (3) sont placés côte à côte.

5. Système de stockage intelligent pour un référentiel d'échantillons selon la revendication 4, dans lequel l'ensemble de bras d'extraction d'axe X (5) comprend une piste de palier d'axe X (16) et un mécanisme de mouvement d'axe X (17) ;
deux extrémités de la piste de palier d'axe X (16) sont disposées verticalement sur la piste de palier d'axe Y (14) et sont reliées au mécanisme de mouvement d'axe Y (15), le mécanisme de mouvement d'axe X (17) est disposé sur la piste de palier d'axe X (16) et est capable de coulisser le long de la piste de palier d'axe X (16), et le mécanisme de mouvement d'axe X (17) est relié à l'appareil de saisie (7) et est capable d'entraîner l'appareil de saisie (7) à se déplacer dans une direction d'axe X ; et
l'axe X est parallèle à une direction le long de laquelle les réservoirs de stockage (3) sont placés côte à côte.

6. Système de stockage intelligent pour un référentiel d'échantillons, comprenant :
deux modules de stockage (1), chaque module de stockage (1) comprenant au moins deux réservoirs de stockage (3) agencés côte à côte, chaque réservoir de stockage (3) est pourvu d'un port d'accès (4) dans une surface d'extrémité supérieure, et les ports d'accès (4) de deux réservoirs de stockage (3) sont opposés et proches l'un de l'autre ; et
un module d'extraction (2) disposé au-dessus des modules de stockage (1) et configuré pour extraire ou stocker des tubes de cryoconservation et/ou des racks de stockage dans les réservoirs de stockage (3) à travers les ports d'accès (4) ;
dans lequel chaque module de stockage (1) est pourvu d'un boîtier de protection de module (26), le module d'extraction (2) est agencé à l'intérieur des boîtiers de protection de module (26) ;
dans lequel le module d'extraction (2) comprend un ensemble de bras d'extraction d'axe X (5), un ensemble de bras d'extraction d'axe Y (6) et un appareil de saisie (7), dans lequel
l'appareil de saisie (7) est configuré pour saisir ou aspirer un tube de cryoconservation et/ou un rack de stockage ;
l'ensemble de bras d'extraction d'axe Y (6) comprend une piste de palier d'axe Y (14) et un mécanisme de mouvement d'axe Y (15), le mécanisme de mouvement d'axe Y (15) est mobile le long de la piste de palier d'axe Y (14) et relié à l'ensemble de bras d'extraction d'axe X (5) pour entraîner l'ensemble de bras d'extraction d'axe X (5) à se déplacer dans une direction d'axe Y ; et
l'ensemble de bras d'extraction d'axe X (5) comprend une piste de coulissement d'axe X (18) et un mécanisme de coulissement (19), le mécanisme de coulissement (19) est mobile le long de la piste de coulissement d'axe X (18) et relié à l'appareil de saisie (7) ; dans lequel la piste de coulissement d'axe X (18) s'étend à travers et entre les boîtiers de protection de module (26) et relie les modules de stockage (1) de sorte que l'appareil de saisie (7) est mobile entre les modules de stockage (1) au moyen de la piste de coulissement d'axe X (18), pour permettre au module d'extraction (2) d'accéder aux réservoirs de stockage de différents modules de stockage (1) ;
dans lequel un canal de transfert pénétrant (8) pénètre à travers les boîtiers de protection de module (26) et est prévu latéralement le long des modules de stockage (1) et est configuré pour transférer un réservoir de transport (31) contenant de l'azote liquide ; et
dans lequel le canal de transfert pénétrant (8) comprend un élément de transmission (9) pour une transmission à travers le canal de transfert pénétrant, pourvu d'une pluralité de canaux de levage (10), et un mécanisme de levage (11) est disposé entre deux réservoirs de stockage (3) de chaque module de stockage (1) et configuré pour lever le réservoir de transport (31) à travers les canaux de levage (10).

7. Système de stockage intelligent pour un référentiel d'échantillons selon la revendication 6, dans lequel le mécanisme de coulissement (19) comprend un ensemble de roues (20), un arbre rotatif de connexion (21) et un support de palier de piste de coulissement (22) ; et
l'ensemble de roues (20) est disposé symétriquement dans la piste de coulissement d'axe X (18) et est capable de glisser le long de la piste de coulissement d'axe X (18), le support de palier de piste de coulissement (22) est disposé sur la piste de coulissement d'axe X (18) et est connecté à l'ensemble de roues (20), et l'arbre rotatif de connexion (21) est disposé sur le support de palier de piste de coulissement (22) et est connecté à l'appareil de saisie (7).

8. Système de stockage intelligent pour un référentiel d'échantillons selon la revendication 5, 6 ou 7, dans lequel l'appareil de saisie (7) comprend un mécanisme d'extraction de tube de cryoconservation (23), un mécanisme de saisie de rack de stockage (24) et un mécanisme de connexion (25) ;
une extrémité supérieure du mécanisme de connexion (25) est connectée à l'ensemble de bras d'extraction d'axe X (5), et une extrémité inférieure du mécanisme de connexion (25) est connectée au mécanisme de saisie de rack de stockage (24) et au mécanisme d'extraction de tube de cryoconservation (23) ; et
l'ensemble de bras d'extraction d'axe X (5) est capable de saisir un tube de cryoconservation ou un rack de stockage dans le réservoir de stockage et est capable d'entraîner le tube de cryoconservation ou le rack de stockage à se déplacer le long de l'axe X, et l'ensemble de bras d'extraction d'axe Y (6) est capable de permettre la saisie d'un tube de cryoconservation ou d'un rack de stockage dans le réservoir de stockage et est capable d'entraîner le tube de cryoconservation ou le rack de stockage à se déplacer le long de l'axe Y.

9. Système de stockage intelligent pour un référentiel d'échantillons selon la revendication 8, dans lequel chacun des modules de stockage (1) comprend en outre un assemblage de vanne (28), et l'assemblage de vanne (28) est disposé sur une surface latérale du boîtier de protection de module (26).

10. Système de stockage intelligent pour un référentiel d'échantillons selon la revendication 9, dans lequel l'assemblage de vanne (28) comprend un mécanisme d'addition de liquide de bobine (29), et le mécanisme d'addition de liquide de bobine (29) est disposé sur une surface latérale du boîtier de protection de module (26).
